# EUROPÄISCHE PATENTSCHRIFT

(11) **EP 0 955 308 B1**
(45) Veröffentlichungstag und Bekanntmachung des Hinweises auf die Patenterteilung: **02.05.2003**
(21) Anmeldenummer: 99105639.1
(22) Anmeldetag: 19.03.1999
(51) Int. Cl.: C07K 1/12, C07K 1/36, C07K 7/23

(54) **Verfahren zur einstufigen Umsalzung und Aufreinigung von Oligopeptiden**
Process for one-step re-salting and purification of peptides
Procédé de résalification et purification de peptides en une seule étape

(30) Priorität: 27.03.1998 DE 19813849
(43) Veröffentlichungstag der Anmeldung: 10.11.1999
(73) Patentinhaber: Degussa AG, 40474 Düsseldorf (DE)
(72) Erfinder: Günther, Kurt, Dr., 63526 Erlensee (DE); Kunz, Franz-Rudolf, Dr., 63526 Erlensee (DE); Drauz, Karlheinz, Prof. Dr., 63579 Freigericht (DE); Müller, Thomas, Dr., 63486 Bruchköbel (DE)

(56) Entgegenhaltungen:
- GB-A- 2 152 059
- US-A- 4 530 920
- CHEMICAL ABSTRACTS, vol. 112, no. 23, 4. Juni 1990 (1990-06-04) Columbus, Ohio, US; abstract no. 217553, J MICHALSKY ET AL.: "Preparation of alpha-aspartylphenylalanine methyl and ethyl esters" XP002109980 & CS 261 262 A (MICHALSKY ET AL.)
- CHEMICAL ABSTRACTS, vol. 118, no. 11, 15. März 1993 (1993-03-15) Columbus, Ohio, US; abstract no. 102429, H NAHARISSOA ET AL.: "Use of 6M hydrochloric acid for removal of the N-alpha-tert-butyloxycarbonyl group during solid-phase peptide synthesis" XP002109981 & PEPT RES, Bd. 5, Nr. 5, 1992, Seiten 293-299,
- CHEMICAL ABSTRACTS, vol. 111, no. 25, 18. Dezember 1989 (1989-12-18) Columbus, Ohio, US; abstract no. 233550, Y KAWASAKI ET AL.: "Development for the simple synthetic method of oligopeptides" XP002109982 & CHEM. EXPRESS, Bd. 3, Nr. 11, 1988, Seiten 703-706,

## Beschreibung

Die vorliegende Erfindung betrifft ein einstufiges Verfahren zur Umsalzung und Aufreinigung von Oligopeptiden.

Oligopeptide entfalten häufig bioaktive Wirkung und erfahren deshalb Anwendungen als Therapeutika. Als Beispiel seien Agonisten und Antagonisten des LHRH genannt, welche unter anderem zur Bekämpfung von bestimmten Krebserkrankungen zum Einsatz kommen.

Die Herstellung der zu reinigenden Oligopeptide kann nach Verfahren des Standes der Technik erfolgen. Sinnvoll sind u.a. die Merrifield-Peptidsynthese an festen Trägermaterialien oder die klassische Synthese in Lösung. Sowohl bei der Merrifield-Festphasensynthese wie auch bei der Synthese in Lösung ist es essentiell, bestimmte Bereiche im Molekül mit Schutzgruppen zu versehen, die am Ende der Herstellung wieder abgespalten werden. Bei der Festphasensynthese ist es darüberhinaus notwendig, das Oligopeptid von dem festen Träger zu entfernen. Für weitere Ausführungen zur Synthese von Peptiden sei auf die einschlägig bekannte Literatur verwiesen (Houben-Weyl, Methoden der organischen Chemie, Band 15/1 und 15/2; M. Bodanszky, Priciples of Peptide Synthesis, Springer Verlag 1984).

Handelt es sich bei dem herzustellenden Peptid um ein Pharmakum, so ist es häufig gewünscht, das Oligopeptid in der Form seines Acetatsalzes vorliegen zu haben, um dem Patienten keine körperfremden oder weitere kritische Stoffe im Gleichschritt mit der Gabe des Medikaments applizieren zu müssen.

Häufig ist es so, daß das Oligopeptid durch Syntheseumstände bedingt nicht als Acetatsalz anfällt, sei es, weil andere Säuren als Essigsäure zur finalen Abspaltung der Schutzgruppen herangezogen werden müssen, sei es, weil die freie Form des Peptids nicht oder nur umständlich herzustellen ist und eine einfache Umwandlung in das Acetat mittels Essigsäure nicht gelingt. Zur Abspaltung der Schutzgruppen oder zur Abspaltung des Peptids von dem zur Synthese benötigten Harz ist man meist auf stärkere Säuren wie Trifluoressigsäure, Salzsäure oder Bromwasserstoff angewiesen. Für weitere Details diese Abspaltungen betreffend sei wiederum auf die Lehrbücher verwiesen (Houben-Weyl, Methoden der organischen Chemie, Band 15/1 und 15/2; M. Bodanszky, Priciples of Peptide Synthesis, Springer Verlag 1984).

In Peptide Research 1992, 5(5), 293 - 299 wird zum Beispiel die Abspaltung von Schutzgruppen mittels HCl beschrieben.

Zur Herstellung eines für die Applikation am Tier oder Menschen benötigten Acetats des betreffenden Oligopeptids ist man in oben genannten Fällen gezwungen, das Oligopeptid umzusalzen.

In GB2152059 werden Oligopeptide durch die Merrifield-Peptidsynthese hergestellt. Das Abspalten der Oligopeptide vom Harz erfolgt dabei mittels HF, welches jedoch vor der auschließenden chromatographischen Aufreinigung mit acetathaltigen Laufmitteln im Stickstoffstrom entfernt wird.

Eine Umsetzung findet hier mithin zumindest nicht einstufig statt.

Das als Wirksubstanz zu prüfende oder als Therapeutikum bereits im Handel befindliche Oligopeptid muß hinsichtlich seiner Reinheit besonderen Anforderungen genügen. Es wird meist in Ermangelung einer geeigneten klasischen Reinigungsmethode eine Aufreinigung des Produktgemisches der Synthese mittels Chromatographie - speziell Hochdruckflüssigkeitschromatographie - angewandt. Dazu ist es notwendig, das Oligopeptid vor dem Aufbringen auf die Säule bevorzugt im Lösungsmittelgemisch des als Eluent gewählten Laufmittels aufzunehmen.

Für Oligopeptide sind in der Literatur bisher mehrere Verfahren beschrieben worden, die deren Umsalzung und Aufreinigung zum Gegenstand haben. Laut Gabriel (Int. J. Peptide Protein Res. 1987, 30, 40-43) kann das Oligopeptid GRF(1-44)-NH₂ aus seinem Trifluoracetat mittels Hochdruckflüssigkeitschromatographie unter Verwendung von pyridin- und essigsäurehaltigen Laufmitteln in das Acetat verwandelt werden. Bezüglich der Reste von Pyridin, welche unweigerlich nach einer solchen Prozedur im Oligopeptid zurückbleiben, bestehen allerdings hinsichtlich toxikologischer Eigenschaften dieses Stoffes Bedenken. Auch aus Arbeitsschutzgründen scheint ein Aufreinigungsverfahren, welches mit größeren Mengen an gefährlichem Pyridn arbeitet, nachteilig.

Unter Umgehung des pyridinhaltigen Laufmittelsystems versuchten Hoeger et al. (BioChromatography 1987, 2,134-142) GnRH-Peptide umzusalzen und aufzureinigen. Ausgehend von den Fluoridsalzen erfolgt hier eine zweistufige Reversed-Phase-Chromatographie mit Gradientenfahrweise in Triethylammoniumphosphat (TEAP)- und Trifluoracetat(TFA)-Puffern mit Acetonitril als Modifier. Nach Lyophilisation der aufgereinigten Peptidfraktionen schließt sich die Konvertierung zu den Acetatsalzen via Anionenaustauschchromatographie mit verdünnter Essigsäure bzw. Reversed-Phase-Chromatographie im Ammoniumacetat/Acetonitrilgradienten an.

Die EP 0145258 beschreibt u.a. die Aufreinigung von HF-Salzen von Nona- und Dekapeptiden der Gruppe der LHRH-Agonisten. Auch hier erfolgt die Umsalzung getrennt vom Aufreinigungsschritt erst über Anionenaustauschchromatographie mit anschließender Endreinigung an einer octadecylsilanisierten Kieselgelphase mittels eines Eluenten bestehend aus Ammoniumacetat und Acetonitril unter Hochdruckbedingungen.

Aufgabe der vorliegenden Erfindung ist die Angabe eines weiteren Verfahrens zur Umsalzung und Aufreinigung von Oligopeptiden, welches diese beiden Arbeitsschritte in einem vereinigt und welches ohne die Verwendung von Pyridin auskommt.

Diese und nicht näher bezeichnete weitere Aufgaben, welche sich jedoch für den Fachmann in naheliegender Weise aus dem Stand der Technik ergeben, sind Gegenstand des kennzeichnenden Teils des Anspruch 1. Bevorzugte Ausgestaltungen des erfindungsgemäßen Verfahrens sind Gegenstand der auf Anspruch 1 rückbezogenen Unteransprüche.

Dadurch, daß man das umzusalzende bzw. aufzureinigende Oligopeptid als sein Hydrochloridsalz per Flüssigchromatographie mittels eines acetathaltigen Laufmittels reinigt, gelangt man äußerst einfach und dennoch vorteilhaft zu praktisch chloridfreien aufgereinigten Oligopetidacetaten. Es gelingt demnach mit dem erfindungsgemäßen Verfahren die vormals nur in zwei Stufen oder durch Einsatz von toxikologisch bedenklichem Pyridin zu bewerkstelligende Aufreinigung und Umsalzung der betrachteten Oligopeptide in einem einzigen Arbeitschritt ohne Zusatz von Pyridin zu vereinen. Die durch das vorliegende Verfahren erhältlichen Produktfraktionen werden vorteilhafterweise vereinigt und durch Lyophilisation getrocknet. Man erhält das Acetat aus dem Chlorid in einer Ausbeute von ca. 85%. Das so erhaltene bis zu einem Prozentsatz von 99,5% reine Oligopeptidacetat kann als Wirkstoff nach Formulierung für eine medikamentöse Therapie herangezogen werden.

Für das vorliegende Verfahren ist es notwendig, daß die Oligopeptide in Form ihrer Chloride zum Einsatz kommen. Der einfachste Weg, um dies zu erreichen, ist der Einsatz von Salzsäure für die Schutzgruppenabspaltung. Auf der anderen Seite sind aber die Peptidhydrolyse und andere Nebenreaktionen an Seitenkettenfunktionalitäten durch die Säurestärke des Abspaltungsagenz bestimmte ungewollte Konkurrenzreaktionen. Aus diesen und anderen Gründen (wie z.B. Löslichkeit des Peptids in TFA) werden für derartige Entschützungen häufig weniger starke wasserfreie Säuren wie z.B. Trifluoressigsäure oder wasserfreie starke Säuremischungen herangezogen wie z.B HBr/Eisessig.

Man kann geschützte Oligopeptide auch mit konzentrierter wäßriger Salzsäure entschützen und die daraus entstehenden Salze der Oligopeptide weisen gegenüber der gängigeren Abspaltung mit der wasserfreien weniger starken Trifluoressigsäure evt. im Gemisch mit organischen Lösungsmitteln oder dem System HBr/Eisessig einen deutlich geringeren Anteil an Nebenprodukten auf.

Bei der eben beschriebenen Abspaltung der Schutzgruppen vom Oligopeptid arbeitet man bevorzugt in einem Temperaturbereich von -25 bis 30°C, besonders bevorzugt sind -10 bis 10°C und ganz besonders bevorzugt sind 0 bis 5°C.

Das Chloridsalz des Oligopeptids kann für die Aufreinigung mittels Flüssigchromatographie als seine konzentrierte salzsaure wäßrige Lösung eingesetzt werden. Bevorzugt wird man aber das Chloridsalz nach der Entschützung isolieren, z.B. durch Lyophilisation, und anschließend im Laufmittelsystem der Flüssigchromatographie oder in Essigsäure zuerst auflösen und auf die Säule geben.

Als Methode der Flüssigchromatographie wird bevorzugt die Hochdruckflüssigkeitschromatographie zur Aufreinigung der Oligopeptide angewandt. Als Lösungsmittelgemische für die Gradientenelution benutzt man Laufmittel nachstehender Zusammensetzung:

| Laufmittel A | | Laufmittel B | |
|---|---|---|---|
| i. | 85 bis 98% Wasser | i. | 20 bis 48% Wasser |
| ii. | 2 bis 10% Essigsäure | ii. | 2 bis 10% Essigsäure |
| iii. | 0 bis 5% Acetonitril | iii. | 50 bis 70% Acetonitril |

oder

| Laufmittel A | | Laufmittel B | |
|---|---|---|---|
| i. | 85 bis 98% Wasser | i. | 0 bis 10% Wasser |
| ii. | 2 bis 10% Essigsäure | ii. | 2 bis 10% Essigsäure |
| iii.0 | bis 5% Methanol | iii.80 | bis 98% Methanol, |

bevorzugt für die Gradientenfahrweise ist ein Lösungsmittelgemisch bestehend aus

| Laufmittel A | Laufmittel B |
|---|---|
| i. 92% Wasser | i. 28% Wasser |
| ii. 5% Essigsäure | ii. 5% Essigsäure |
| iii.3% Acetonitril | iii.67% Acetonitril |

oder

| Laufmittel A | Laufmittel B |
|---|---|
| i. 90% Wasser | i. 5% Wasser |
| ii. 5% Essigsäure | ii. 5% Essigsäure |
| iii.5% Acetonitril | iii.90% Acetonitril |

Die Aufreinigung erfolgt bevorzugt bei einer Säulentemperatur von 5 bis 50°C, besonders bevorzugt sind 15 bis 35°C und ganz besonders bevorzugt sind 20 bis 30°C. Der Säulendruck sollte zwischen 5 und 100 bar, bevorzugt zwischen 20 und 80 bar und besonders bevorzugt zwischen 30 und 60 bar betragen. Als stationäre Phase können prinzipiell alle dem Fachmann geläufigen Materialien zur Reinigung genommen werden.Besonders gut eignet sich ein Reversed-Phase-Material. Als Reversed-Phase-Material werden Säulenpackungen verstanden die auf Trägermaterialien wie Kieselgel oder organischem Polymer basieren. Im Falle von Kieselgelen können die hydrophilen Oberflächen durch Organosilane modifiziert sein. Hierzu eignet sich neben anderen C-2, C-8 oder C-18 Modifikationen. Besonders bevorzugt ist der Einsatz einer C-18 modifizierten RP-18 Phase, ganz besonders bevorzugt ist die Nucleosil® 300-7-C₁₈ der Firma Macherey&Nagel oder Purospher® RP 18 (10µm) der Firma Merck.

Als Oligopeptide werden in der vorliegenden Anmeldung Peptide mit fünf bis fünfundzwanzig Aminosäuren verstanden. Bevorzugt ist der Bereich von Oligopeptiden mit acht bis zwölf Aminosäuren. Ganz besonders bevorzugt kommen Oligopeptide mit zehn Aminosäuren zur Anwendung.

Die oben beschriebene flüssigchromatographische Methode zur Umsalzung und Aufreinigung der Oligopeptide läßt sich sowohl nach der sogenannten Simulated-Moving-Bed-Technik als auch mittels cyclischer Chromatographie durchführen .

Ganz außerordentlich vorteilhaft kommt das erfindungsgemäße Verfahren in einer Synthese zur Herstellung der LHRH-Antagonisten Cetrorelix (1) und Antarelix (2) 
Ac-D-Nal(2)-D-Phe(4Cl)-D-Pal(3)-Ser-Tyr-D-Cit-Leu-Arg-Pro-
D-Ala-NH2 · 2CH3COOH

1
 
Ac-D-Nal(2)-D-Phe(4Cl)-D-Pal(3)-Ser-Tyr-D-Hci-Leu-Lys(ε-
isopropyl)-Pro-D-Ala-NH2 · 2CH3COOH
2
 zum Einsatz. Die Einführung von tert.-Butylschutzgruppen in der Seitenkette von Serin und Tyrosin hat sich bei der Synthese besonders bewährt. Zur Gewinnung des Endproduktes müssen diese Schutzgruppen unter sauren Bedingungen (TFA bzw. HCl) wieder abgespalten werden. Bei der Abspaltung der tert.-Butylgruppen mit Salzsäure bilden sich deutlich weniger Nebenprodukte als bei der Verwendung von TFA.

Das optimierte Trennverfahren ist geeignet für die Prozeßchromatographie und erlaubt den Einsatz von präparativen HPLC-Säulen mit Innendurchmessern von größer 30 cm und Injektionsmengen von mehr als 200 g pro Chromatographielauf.

Damit ist das vorliegende Verfahren mit ursächlich dafür, daß z.B. oben genannte Oligopeptide in vorteilhafter und damit ökonomisch günstigerer Weise im Multikilogrammaßstab bereitzustellen sind.

Die Ausgangssubstanzen für die hier beschriebene Erfindung lassen sich nach dem Fachmann an sich bekannten Methoden herstellen. Es sei auf die einschlägigen Lehrbücher verwiesen (Houben-Weyl, Methoden der organischen Chemie, Band 15/1 und 15/2; M. Bodanszky, Priciples of Peptide Synthesis, Springer Verlag 1984).

### Beispiel 1:

### Darstellung von Ac-D-Mal-D-p-Cl-Phe-D-Pal-Ser-Tyr-D-Cit-Leu-Arg-Pro-D-Ala-NH₂ x 2HCl (1a) (Cetrorelix-Hydrochlorid)

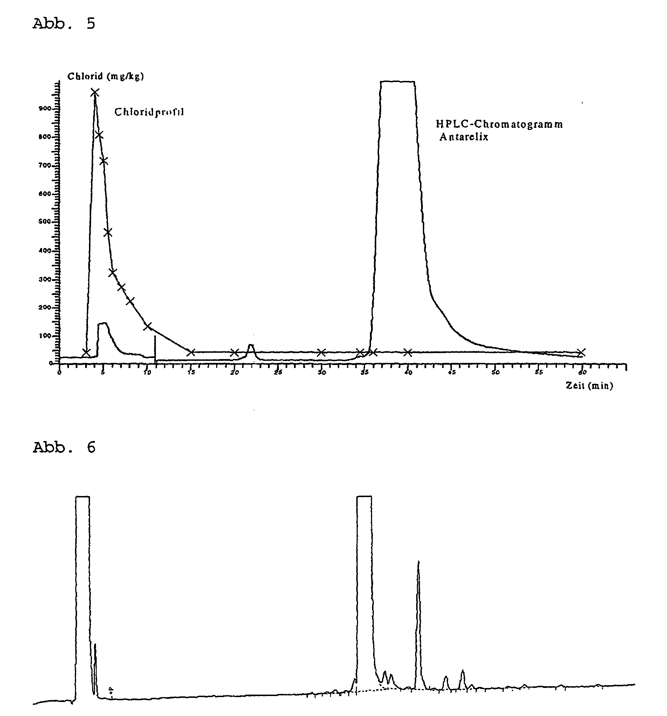

50 g (31.65 mmol) ) Ac-D-Nal-D-p-Cl-Phe-D-Pal-Ser(^{t}Bu)-Tyr(^{t}Bu)-D-Cit-Leu-Arg(HCl)-Pro-D-Ala-NH₂ werden unter starkem Rühren in 200 ml eisgekühlte konzentrierte Salzsäure gegeben. Man rührt ca. 1h bei 0-5°C, gibt die Reaktionsmischung auf ein gerührtes Gemisch aus 0.75 l n-Butanol und 0.5 kg Eis, trennt nach Zugabe von 120 ml Wasser die Phasen, stellte den pH-Wert der organischen Phase mit Natronlauge auf ca. 2 ein und dampft die butanolische Lösung i.Vak. ein. Der Rückstand wird in 0.5 l tert-Butylmethylether suspendiert, abgesaugt, mit 0.5 l tert-Butylmethylether gewaschen und i.Vak. getrocknet. Ausbeute an 1a : 49 g (103 %, Substanz enthält ca. 4 Gew.-% NaCl), HPLC-Reinheit 94.4 Fl.-%. (siehe Abb. 6)

### Beispie12:

### Darstellung von Ac-D-Nal-D-p-Cl-Phe-D-Pal-Ser-Tyr-D-Cit-Leu-Arg-Pro-D-Ala-NH₂ x 2TFA (1b) (Cetrorelix-Trifluoracetat)

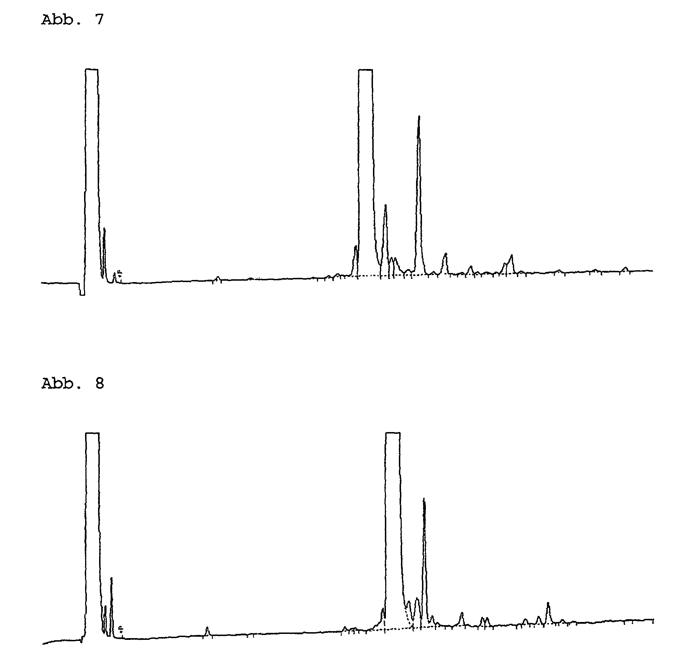

1 g (0.633 mmol) Ac-D-Nal-D-p-Cl-Phe-D-Pal-Ser(^{t}Bu)-Tyr(^{t}Bu)-D-Cit-Leu-Arg(HCl)-Pro-D-Ala-NH₂ werden in 10 ml Trifluoressigsäure gelöst, die Lösung 1.5 h gerührt und anschließend in 100 ml eiskalten Diisopropylether gegeben. Das Produkt 1b wird abgesaugt, mit Diisopropylether gewaschen und i.Vak. getrocknet. Ausbeute an 1b : 1.05 g (100 %), HPLC-Reinheit 89.6 Fl.-%. (siehe Abb. 7)

### Beispiel 3:

### Darstellung von Ac-D-Nal-D-p-Cl-Phe-D-Pal-Ser-Tyr-D-Hci-Leu-Lys(ε-isopropyl)-Pro-D-Ala-NH₂ x 2HCl (2a) (Antarelix-Hydrochlorid)

77.3 g (46.2 mmol) Ac-D-Nal-D-p-Cl-Phe-D-Pal-Ser(^{t}Bu)-Tyr(^{t}Bu)-D-Hci-Leu-Lys(ε-Boc)(ε-isopropyl)-Pro-D-Ala-NH₂ werden unter starkem Rühren in 400 ml eisgekühlte, konzentrierte Salzsäure gegeben. Nach ca. 1 h gießt man die Reaktionsmischung auf ein Gemisch aus 0.85 l Wasser/0.85 kg Eis, extrahiert die wäßrige Lösung zweimal mit je 0.9 l n-Butanol, stellt den pH-Wert der vereinigten Butanolphasen mit gesättigter, wäßriger Natriumhydrogencarbonatlösung auf ca. 2 ein, trennt die Phasen und dampft die organische Phase i.Vak. ein. Der Rückstand wird mit 2 l tert-Butylmethylether digeriert, abgesaugt, mit tert-Butylmethylether gewaschen und i.Vak. getrocknet. Ausbeute 72 g (104 %, Substanz enthält ca. 2 Gew.-% NaCl und Reste Butanol), HPLC-Reinheit 94.0%. (siehe Abb. 8)

### Beispiel 4:

### Darstellung von Ac-D-Nal-D-p-Cl-Phe-D-Pal-Ser-Tyr-D-Hci-Leu-Lys(ε-isopropyl)-Pro-D-Ala-NH₂ x 2TFA (2b) (Antarelix-Trifluoracetat)

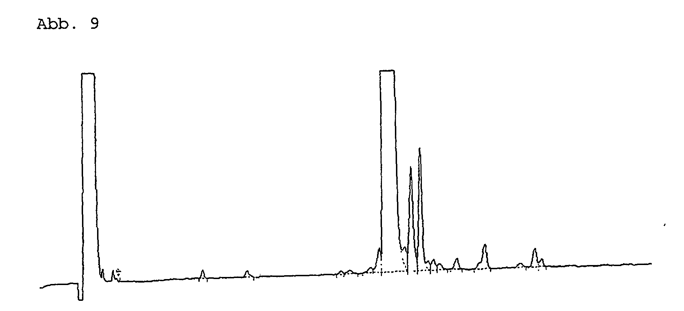

1 g (0.6 mmol) Ac-D-Nal-D-p-Cl-Phe-D-Pal-Ser(^{t}Bu)-Tyr(^{t}Bu)-D-Hci-Leu-Lys(ε-Boc)(ε-isopropyl)-Pro-D-Ala-NH₂ werden in 10 ml Trifluoressigsäure gelöst, die Lösung ca. 1.5 h gerührt und anschließend in 100 ml eiskalten Diisopropylether gegeben. Das Produkt 1b wird abgesaugt, mit Diisopropylether gewaschen und i.Vak. getrocknet. Ausbeute an 1b : 1.01 g (100 %), HPLC-Reinheit 89.4 Fl.-%. (siehe Abb. 9)

### Beispiel 5:

### Präparative Aufreinigung von Cetrorelix aus dem Rohprodukt der klassischen Synthese in Lösung (Beispiel 1):

18 g des Rohproduktes aus einem Verfahren des Beispiels 1 werden in 500 ml 30%-iger Essigsäure gelöst und nach Filtration (über Seitz K-700-Filter) auf die Säule (Länge 250 mm, Innendurchmesser 100 mm) appliziert. Als stationäre Phase kann alternativ Nucleosil 300-7-C₁₈ der Fa. Macherey & Nagel oder Purospher RP 18 (10 mm) der Fa. Merck eingesetzt werden. Dabei wird die Säule zunächst 20 Minuten mit einem Lösungsmittelgemisch aus 95 % mobiler Phase A (970 ml Reinstwasser + 30 ml Acetonitril + 50 ml 100 %-iger Essigsäure) und 5 % mobiler Phase B (700 ml Acetonitril + 300 ml Reinstwasser + 50 ml 100 %-iger Essigsäure) konditioniert. Anschließend erfolgt die Chromatographie auf der Nucleosil Phase nach folgendem Gradientenprogramm:

| Zeit (min) | Konzentration A (Vol %) | Konzentration B (Vol %) |
|---|---|---|
| 0 | 95 | 5 |
| 9 | 95 | 5 |
| 10 | 77 | 23 |
| 22 | 77 | 23 |
| 37 | 67 | 33 |
| 47 | 0 | 100 |
| 55 | 0 | 100 |

Der Eluentenfluß beträgt 200 ml/min, wobei sich je nach Gradientenbedingungen ein Säulendruck von 38-60 bar aufbaut.

### Alternativ erfolgt die Chromatographie auf dem Purospher-Träger nach folgendem Gradientenprogramm :

| Zeit (min) | Konzentration A (Vol %) | Konzentration B (Vol %) |
|---|---|---|
| 0 | 95 | 5 |
| 20 | 95 | 5 |
| 21 | 70 | 30 |
| 60 | 65 | 35 |
| 61 | 0 | 100 |
| 70 | 0 | 100 |

Der Eluentenfluß beträgt in diesem Fall 300 ml/min, wobei sich je nach Gradientenbedingungen ein Säulendruck von 35-50 bar aufbaut.

Die Peakdetektion erfolgt im UV bei 270 nm, wobei eine manuelle Fraktionierung vorgenommen wird. Abgetrennt vom Hauptpeak (Reinheit > 99.5 %) werden ansteigende und abfallende Flanken (Reinheit ca. 95 %), die rezykliert werden. Aus den Fraktionen wird am Rotationsverdampfer bei ca. 50°C und Wasserstrahlvakuum Acetonitril bis auf ca. 1% entfernt. Anschliessend erfolgt die Lyophilisation der eingeengten Eluate.

### HPLC-Analytik des eingesetzten Rohproduktes:

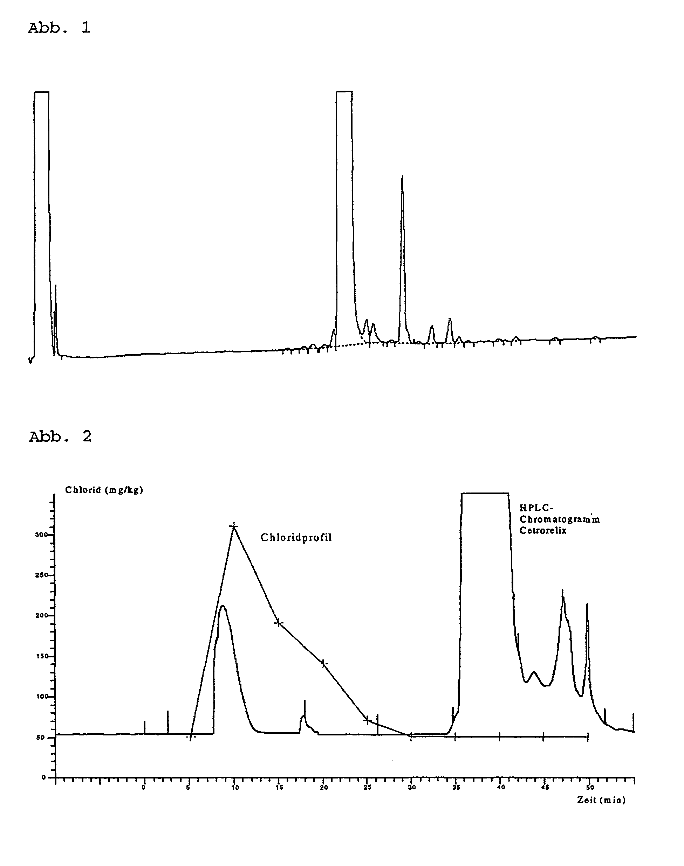

Dargestellt in Abbildung 1.

### Spezifikation des Rohproduktes:

- Peptidreinheit :: 94.4 % Fläche
- Chloridgehalt :: 6.2 %

### Präparative HPLC des Cetrorelix-Syntheserohproduktes auf der Nucleosil-Phase:

Dargestellt in Abbildung 2.

### Präparative HPLC des Cetrorelix-Syntheserohproduktes auf dem Purospher-Träger:

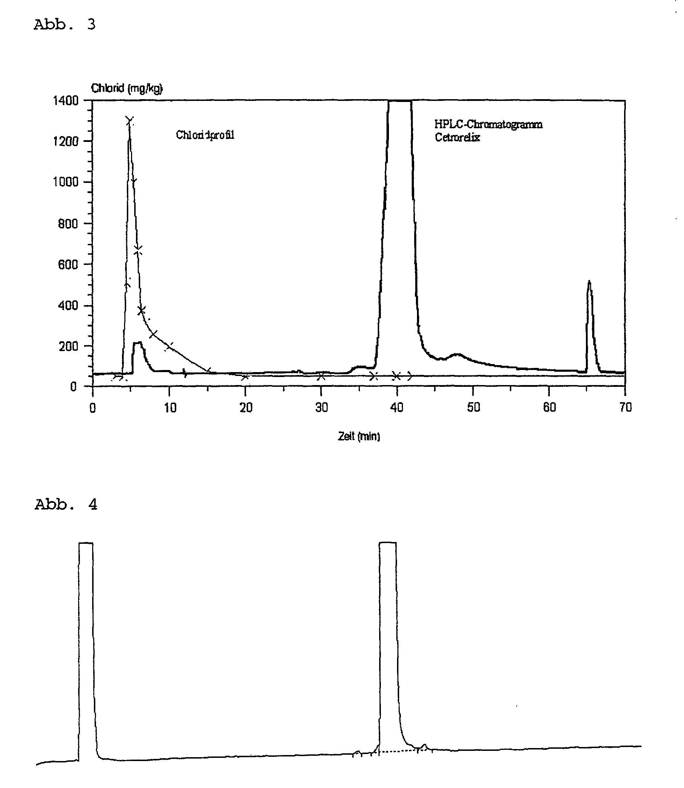

Dargestellt in Abbildung 3.

### HPLC-Chromatogramm des aufgereinigten Endproduktes:

Dargestellt in Abbildung 4.

### Spezifikation des Endproduktes

- Peptidreinheit :: 99.75 %
- Chloridgehalt :: 220 ppm
- Acetatgehalt: 6.5 %

### Beispiel 6:

### Präparative Aufreinigung von Antarelix aus dem Rohprodukt der klassischen Synthese in Lösung

15 g des Rohproduktes werden in 500 ml 30%-iger Essigsäure gelöst und nach Filtration (über Seitz K-700-Filter) auf die Säule (Länge 250 mm, Innendurchmesser 100 mm) appliziert. Als stationäre Phase dient Purospher RP 18 (10 mm) der Fa. Merck. Dabei wird die Säule zunächst 20 Minuten mit einem Lösungsmittelgemisch aus 95 % mobiler Phase A (970 ml Reinstwasser + 30 ml Acetonitril + 50 ml 100 %-iger Essigsäure) und 5 % mobiler Phase B (700 ml Acetonitril + 300 ml Reinstwasser + 50 ml 100 %-iger Essigsäure) konditioniert. Anschließend erfolgt die Chromatographie nach folgendem Gradientenprogramm:

| Zeit (min) | Konzentration A (Vol %) | Konzentration B (Vol %) |
|---|---|---|
| 0 | 95 | 5 |
| 15 | 95 | 5 |
| 16 | 70 | 30 |
| 56 | 65 | 35 |
| 57 | 0 | 100 |
| 65 | 0 | 100 |

Der Eluentenfluß beträgt 300 ml/min, wobei sich je nach Gradientenbedingungen ein Säulendruck von 35-50 bar aufbaut. Die Peakdetektion erfolgt im UV bei 270 nm, wobei eine manuelle Fraktionierung vorgenommen wird. Abgetrennt vom Hauptpeak (Reinheit > 99.5 %) werden ansteigende und abfallende Flanken (Reinheit ca. 95 %), die rezykliert werden. Aus den Fraktionen wird am Rotationsverdampfer bei ca. 50°C und Wasserstrahlvakuum Acetonitril bis auf ca. 1% entfernt. Anschliessend erfolgt die Lyophilisation der eingeengten Eluate.

### Präparative HPLC des Antarelix-Syntheserohproduktes auf dem Purospher-Träger:

Dargestellt in Abbildung 5.

### Spezifikation des Endproduktes

- Peptidreinheit :: 99.39 %
- Chloridgehalt :: <200 ppm
- Acetatgehalt: 7.5 %

### Beispiel 7:

### Präparative Aufreinigung von Cetrorelix im System Methanol/Wasser/Essigsäure

4 g des Rohproduktes werden in 60 ml 30%-iger Essigsäure gelöst und nach Filtration (über Seitz K-700-Filter) auf die Säule (Länge 250 mm, Innendurchmesser 40 mm) appliziert. Als stationäre Phase dient Deltapak 300 Å, 15 mm der Fa. Millipore. Dabei wird die Säule zunächst 20 Minuten mit mobiler Phase A (950 ml Reinstwasser + 50 ml Methanol + 60 ml 100 %-iger Essigsäure) konditioniert.

Anschließend erfolgt die Chromatographie nach folgendem Gradientenprogramm (mobile Phase B: 950 ml Methanol + 50 ml Reinstwasser + 60 ml 100 %-iger Essigsäure):

| Zeit (min) | Konzentration A (Vol %) | Konzentration B (Vol %) |
|---|---|---|
| 0 | 100 | 0 |
| 10 | 100 | 0 |
| 11 | 70 | 30 |
| 40 | 30 | 70 |
| 41 | 0 | 100 |
| 50 | 0 | 100 |

Der Eluentenfluß beträgt 60 ml/min, wobei sich je nach Gradientenbedingungen ein Säulendruck von 20-30 bar aufbaut. Die Peakdetektion erfolgt im UV bei 270 nm, wobei eine manuelle Fraktionierung vorgenommen wird. Abgetrennt vom Hauptpeak (Reinheit > 99.5 %) werden ansteigende und abfallende Flanken (Reinheit ca. 95 %), die rezykliert werden. Aus den Fraktionen wird am Rotationsverdampfer bei ca. 50°C und Wasserstrahlvakuum Methanol bis auf ca. 1% entfernt. Anschliessend erfolgt die Lyophilisation der eingeengten Eluate.

### Spezifikation des Endproduktes

- Peptidreinheit :: 99.60 %
- Chloridgehalt :: 220 ppm
- Acetatgehalt: 7.1 %

## Patentansprüche

1. Verfahren zur Umsalzung und Aufreinigung von Oligopeptiden , **dadurch gekennzeichnet, daß** das Verfahren einstufig durch Flüssigchromatographie mit acetathaltigen Laufmitteln zum Erhalt des Oligopeptidacetats durchgeführt wird, wobei man die Oligopeptide als deren Hydrochloridsalze zur Reinigung einsetzt.

2. Verfahren nach Anspruch 1,
**dadurch gekennzeichnet,**
**daß** man die geschützten Vorstufen der Oligopeptide mit konzentrierter Salzsäure entschützt.

3. Verfahren nach Anspruch 2,
**dadurch gekennzeichnet,**
**daß** man das pure Chloridsalz des Oligopeptids durch Lyophilisation dessen salzsaurer Lösung erhält.

4. Verfahren nach Anspruch 1,
**dadurch gekennzeichnet,**
**daß** man das Chloridsalz des Oligopeptids als seine konzentrierte salzsaure wäßrige Lösung einsetzt.

5. Verfahren nach Anspruch 1 bis 3,
**dadurch gekennzeichnet,**
**daß** man das pure Chloridsalz nach Lösen in dem Laufmittel der Flüssigkeitschromatographie oder nach Lösen in Essigsäure einsetzt.

6. Verfahren nach Anspruch 2,
**dadurch gekennzeichnet,**
**daß** man bei einer Temperatur von -25 bis 30°C arbeitet.

7. Verfahren nach Anspruch 1,
**dadurch gekennzeichnet,**
**daß** man als Flüssigchromatographie die Hochdruckflüssigkeitschromatographie anwendet.

8. Verfahren nach Anspruch 7,
**dadurch gekennzeichnet,**
**daß** man mit einem Lösungsmittelgemisch der folgenden Zusammensetzung arbeitet:
| Laufmittel A | | Laufmittel B | |
|---|---|---|---|
| i. | 85 bis 98% Wasser | i. | 20 bis 48% Wasser |
| ii. | 2 bis 10% Essigsäure | ii. | 2 bis 10% Essigsäure |
| iii. | 0 bis 5% Acetonitril | iii. | 50 bis 70% Acetonitril |
oder
| Laufmittel A | | Laufmittel B | |
|---|---|---|---|
| i. | 85 bis 98% Wasser | i. | 0 bis 10% Wasser |
| ii. | 2 bis 10% Essigsäure | ii. | 2 bis 10% Essigsäure |
| iii. | 0 bis 5% Methanol | iii. | 80 bis 98% Methanol, |

9. Verfahren nach Anspruch 8,
**dadurch gekennzeichnet,**
**daß** man mit folgendem Lösungsmittelgemisch arbeitet:
| Laufmittel A | | Laufmittel B | |
|---|---|---|---|
| i. | 92% Wasser | i. | 28% Wasser |
| ii. | 5% Essigsäure | ii. | 5% Essigsäure |
| iii. | 3% Acetonitril | iii. | 67% Acetonitril |
oder
| Laufmittel A | | Laufmittel B | |
|---|---|---|---|
| i. | 90% Wasser | i. | 5% Wasser |
| ii. | 5% Essigsäure | ii. | 5% Essigsäure |
| iii. | 5% Acetonitril | iii. | 90% Acetonitril |

10. Verfahren nach Anspruch 8 und 9,
**dadurch gekennzeichnet,**
**daß** man bei einer Temperatur von 5 bis 50°C arbeitet.

11. Verfahren nach Anspruch 8 bis 10,
**dadurch gekennzeichnet,**
**daß** man mit einem Druck von 5 bis 100 bar arbeitet.

12. Verfahren nach Anspruch 8 bis 11,
**dadurch gekennzeichnet,**
**daß** man als stationäre Phase Reversed-Phase-Säulenmaterial verwendet.

13. Verfahren nach Anspruch 1,
**dadurch gekennzeichnet,**
**daß** man als Oligopeptid ein Peptid aus mindestens fünf und höchstens fünfundzwanzig Aminosäuren einsetzt.

14. Verfahren nach Anspruch 13,
**dadurch gekennzeichnet,**
**daß** man ein Peptid aus acht bis zwölf Aminosäuren einsetzt.

15. Verfahren nach Anspruch 14,
**dadurch gekennzeichnet,**
**daß** man ein Peptid aus zehn Aminosäuren einsetzt.

16. Verfahren nach Anspruch 1,
**dadurch gekennzeichnet,**
**daß** man die Flüssigchromatographie nach der Simulated-Moving-Bed Methode oder mittels cyclischer Chromatographie durchführt.

## Claims

1. Process for the resalting and purification of oligopeptides, **characterised in that** the process is carried out in one stage by liquid chromatography with acetate-containing solvents in order to obtain the oligopeptide acetate, the oligopeptides being used in the form of their hydrochloride salts for the purification.

2. Process according to claim 1, **characterised in that** the protected precursors of the oligopeptides are deprotected with concentrated hydrochloric acid.

3. Process according to claim 2, **characterised in that** the pure chloride salt of the oligopeptide is obtained by lyophilisation of its hydrochloric acid solution.

4. Process according to claim 1, **characterised in that** the chloride salt of the oligopeptide is used in the form of its concentrated hydrochloric acid aqueous solution.

5. Process according to claims 1 to 3, **characterised in that** the pure chloride salt is used after dissolution in the liquid chromatography solvent or after dissolution in acetic acid.

6. Process according to claim 2, **characterised in that** the process is carried out at a temperature of -25° to 30°C.

7. Process according to claim 1, **characterised in that** high pressure liquid chromatography is used as liquid chromatography.

8. Process according to claim 7, **characterised in that** the chromatography is carried out with a solvent system having the following composition:
| Solvent A | | Solvent B | |
|---|---|---|---|
| i. | 85 to 98 % water | i. | 20 to 48 % water |
| ii. | 2 to 10 % acetic acid | ii. | 2 to 10 % acetic acid |
| iii. | 0 to 5 % acetonitrile | iii. | 50 to 70% acetonitrile |
or
| Solvent A | | Solvent B | |
|---|---|---|---|
| i. | 85 to 98 % water | i. | 0 to 10 % water |
| ii. | 2 to 10 % acetic acid | ii. | 2 to 10 % acetic acid |
| iii. | 0 to 5 % methanol | iii. | 80 to 98 % methanol |

9. Process according to claim 8, **characterised in that** the chromatography is carried out with the following solvent system:
| Solvent A | | Solvent B | |
|---|---|---|---|
| i. | 92 % water | i. | 28 % water |
| ii. | 5 % acetic acid | ii. | 5% acetic acid |
| iii. | 3% acetonitrile | iii. | 67% acetonitrile |
or
| Solvent A | | Solvent B | |
|---|---|---|---|
| i. | 90% water | i. | 5% water |
| ii. | 5 % acetic acid | ii. | 5% acetic acid |
| iii. | 5% acetonitrile | iii. | 90% acetonitrile |

10. Process according to claims 8 and 9, **characterised in that** the process is carried out at a temperature from 5° to 50°C.

11. Process according to claims 8 to 10, **characterised in that** the process is carried out at a pressure of 5 to 100 bar.

12. Process according to claims 8 to 11, **characterised in that** reversed phase column material is used as stationary phase.

13. Process according to claim 1, **characterised in that** a peptide of at least five and at most twenty-five amino acids is used as oligopeptide.

14. Process according to claim 13, **characterised in that** a peptide of eight to twelve amino acids is used.

15. Process according to claim 14, **characterised in that** a peptide of ten amino acids is used.

16. Process according to claim 1, **characterised in that** the liquid chromatography is carried out according to the simulated moving bed method or by means of cyclical chromatography.

## Revendications

1. Procédé de transformation de la nature des sels et de purification d'oligopeptides,
**caractérisé en ce qu'**
on réalise le procédé en une étape au moyen d'une chromatographie en phase liquide avec des éluants contenant de l'acétate pour obtenir de l'acétate d'oligopeptide, opération dans laquelle on utilise les oligopeptides sous la forme de leurs sels de chlorhydrate en vue de la purlflcation.

2. Procédé selon la revendication 1,
**caractérisé en ce qu'**
on déprotège avec de l'acide chlorhydrique concentré les précurseurs protégés des oligopeptides.

3. Procédé selon la revendication 2,
**caractérisé en ce qu'**
on obtient le gel de chlorure pur de l'ollgopeptide par lyophilisation de sa solution dans l'acide chlorhydrique.

4. Procédé selon la revendication 1,
caractérlsé en ce qu'
on utilise le sels de chlorure de l'oligopeptide sous la forme de sa solution aqueuse concentrée dans l'acide chlorhydrique.

5. Procédé selon l'une des revendications 1 à 3,
**caractérisé en ce qu'**
on utilise le sel de chlorure pur après dissolution dans l'éluant de la chromatographie en phase liquide ou après dissolution dans l'acide acétique.

6. Procédé selon la revendication 2,
**caractérisé en ce qu'**
on travaille à une température allant de -25 à 30°C.

7. Procédé selon la revendication 1,
**caractérisé en ce qu'**
on utilise comme chromatographie en phase liquide une chromatographie en phase liquide haute pression.

8. Procédé selon la revendication 7,
**caractérisé en ce qu'**
on travaille avec un mélange de solvants avant la composition suivante :
| Eluant A | Eluant B |
|---|---|
| i. 85 à 98 % d'eau | i. 20 à 48 % d'eau |
| ii. 2 à 10 % d'acide acétique | ii. 2 à 10 % d'acide acétique |
| iii. 0 à 5 % d'acétonitrtie | iii. 50 à 70 % d'acétonitrile |
ou
| Eluant A | Eluant B |
|---|---|
| i. 85 à 98 % d'eau | i. 0 à 10 % d'eau |
| ii. 2 à 10 % d'acide acétique | ii. 2 à 10 % d'acide acétique |
| iii. 0 à 5 % de méthanol | iii. 80 à 98 % de méthanol |

9. Procédé selon la revendication 8.
**caractérisé en ce qu'**
on travaille avec le mélange de solvants suivant :
| Eluant A | Eluant B |
|---|---|
| i. 92 % d'eau | i. 28 % d'eau |
| ii. 5 % d'acide acétique | ii. 5 % d'acide acétique |
| Iii. 3 % d'acétonitrile | iii. 67 % d'acétonitrile |
ou
| Eluant A | Eluant B |
|---|---|
| i. 90 % d'eau | i. 5 % d'eau |
| ii. 5 % d'acide acétique | ii. 5 % d'acide acétique |
| Iii. 5 % d'acétonitrile | iii. 90 % d'acétonitrile |

10. Procédé selon les revendications 8 et 9,
**caractérisé en ce qu'**
on travaille à une température allant de 5 à 50°C.

11. Procédé selon les revendications 8 à 10,
**caractérisé en ce qu'**
on travaille à une pression allant de 5 à 100 bars.

12. Procédé selon l'une des revendications 8 à 11.
**caractérisé en ce qu'**
on utilise comme phase stationnaire un matérlau de colonnes à phases inversées.

13. Procédé selon la revendication 1,
**caractérisé en ce qu'**
on utilise somme peptide un peptide constitué d'au moins 5 et au plus 25 acides aminés.

14. Procédé selon la revendication 13,
**caractérisé en ce qu'**
on utilise un peptide constitué de 8 à 12 acides aminés.

15. Procédé selon la revendication 14,
**caractérisé en ce qu'**
on utilise un peptide constitué de 10 acides aminés.

16. Procédé selon la revendication 1,
**caractérisé en ce qu'**
on effectue la chromatographie en phase liquide selon le procédé du "Simultated-Moving-Bed" à lit mobile simulé ou au moyen d'une chromatographie cyclique.
